# EUROPEAN PATENT APPLICATION

(11) **EP 1 830 188 A1**
(43) Date of publication of application: **05.09.2007**
(21) Application number: 05806658.0
(22) Date of filing: 18.11.2005
(51) Int. Cl.: G01N 33/53, C12Q 1/68, G01N 37/00, G06F 19/00

(54) **METHOD, PROGRAM AND SYSTEM FOR THE STANDARDIZATION OF GENE EXPRESSION AMOUNT**

(30) Priority: 13.12.2004 JP 2004360417
(71) Applicant: Sony Corporation, Minato-ku Tokyo 108-0075 (JP)
(72) Inventor: YASUNORI, Ohto, c/o Sony Corporation, Tokyo 108-0075 (JP); ABE, Tomoteru, c/o Sony Corporation, Tokyo 108-0075 (JP)
(74) Representative: Horner, David Richard
(86) International application number: PCT/JP2005/021277
(87) International publication number: WO 2006/064631

(57) **Abstract**

The present invention aims at presenting novel means for analyzing and correcting gene expression amounts. There is provided a gene expression amount normalizing method in which the number of cells in a sample is obtained by measuring a repeated sequence present in a substantially fixed proportion in a genome contained in the sample, and the number of cells obtained is used as an index for normalizing gene expression amounts obtained from the same sample. For example, a DNA sample 33 and an RNA sample 34 are obtained from the same sample 32, the DNA sample 33 is used as a sample for obtaining the number of cells, and the RNA sample 34 is used as a sample for obtaining the gene expression amounts, whereby the number of cells contained in the sample 32 and the gene expression amounts relating to the same sample 32 can be obtained. Therefore, by converting the gene expression amounts to values per a fixed number of cells, the gene expression amounts can be normalized to values which can be compared with those obtained by other gene expression analyses.

## Description

### Technical Field

The present invention relates to a technical field relating to the normalization or standardization, analysis, and correction of gene expression amount measurement data obtained by use of a bioassay substrate such as a DNA chip.

### Background Art

In recent years, putting DNA chips or DNA microarrays (hereinafter referred to as "DNA chips" in the present invention) into practical use has been progressing. A DNA chip has a multiplicity and many kinds of DNA oligo-strands which are integrately immobilized on a substrate surface as probe nucleic acids. By use of the DNA chip, the hybridizations between the probe nucleic acids immobilized on the substrate surface and target nucleic acids in sample nucleic acids sampled from cells or the like are detected, whereby the gene expressions in the sample cells can be all-inclusively analyzed.

Along with the enhancement of the hybridization detecting technology in the gene expression analysis using DNA chips, not only the simple detection of the presence or absence of gene expressions but also quantitative measurement of gene expression amounts have been coming to be possible. For example, the technology of obtaining quantitative numerical values indicative of the gene expression amounts by quantitative measurement of fluorescent intensity in detecting the hybridization has been partly put to practical use.

In such a situation, trials have been made to achieve normalization of the quantitative numerical values indicating the gene expression amounts. The term "normalization" used here means conversion of the quantitative numerical values into numerical values which can be compared with gene expression amounts obtained by other gene expression analyses. As a method for normalizing gene expression amounts, for example, there has been proposed a method in which the gene expression amount of a gene being steadily expressed is used as an index for normalization of gene expression amounts.

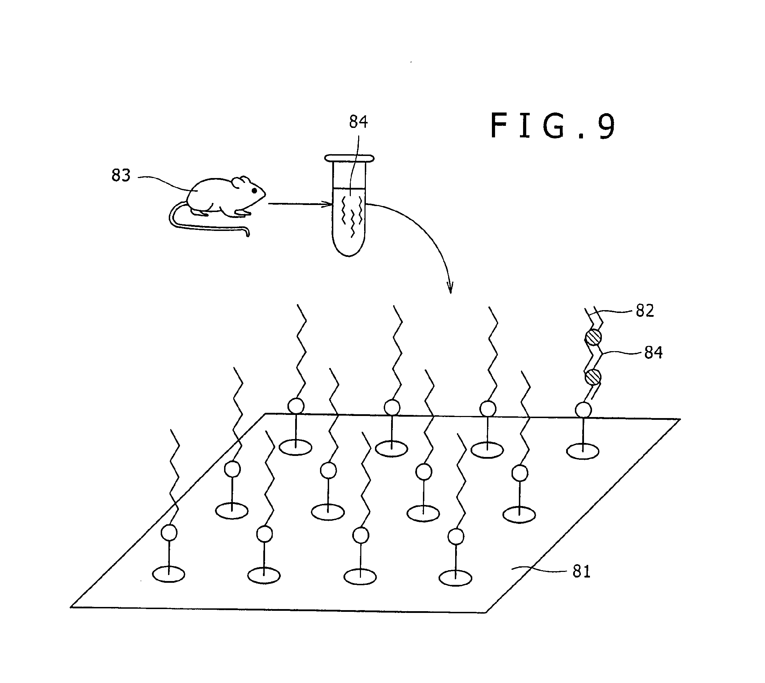

The method in which the gene expression amount of a gene expressed steadily is used as an index for the normalization will be described below, referring to FIG. 9. As shown in FIG. 9, a probe nucleic acid 82 capable of hybridization with a gene expressed steadily is preliminarily immobilized on a substrate surface 81 of a DNA chip. Then, the amount of hybridization between the probe nucleic acid 82 and a sample nucleic acid 84 sampled from an individual 83 served to the gene expression analysis is detected through fluorescent intensity or the like, whereby the gene expression amount of the gene in the individual 83 served to the gene expression analysis is obtained, and is used as an index for normalization.

Other than the above, the preceding references relating to the analyzing method, correcting method, and the like for gene expression amounts obtained by use of DNA chips or the like include, for example, Japanese Patent Laid-open Nos. 2002-71688, 2002-267668, and 2003-28862.

The method of using the gene expression amount of a gene expressed steadily as an index for normalization has had the problem that it is difficult to search for a gene steadily expressed at a fixed value. In practice, the gene expression amount is varied in many cases depending on the time when the cells are sampled, an external stress exerted on the cells, or the like factors. In addition, where the gene expression amount of the gene expressed steadily is used as an index for normalization, it has been difficult to decide whether the variation in the gene expression amount is due to the above-mentioned reason or due to variation in the number of cells used for preparation of the sample.

Therefore, where gene expression amounts are normalized by use as an index therefor the gene expression amount of the gene expressed steadily, there has been a large dispersion of each of the normalized numerical values. Besides, since the dispersion arises from a combined cause, it has been difficult to correct the numerical values before use.

Accordingly, it is a primary object of the present invention to present novel means for analyzing and correcting gene expression amounts and to enhance the accuracy of normalization of gene expression amounts.

### Disclosure of Invention

According to the present invention, there is provided a method of normalizing a gene expression amount, including the steps of: measuring a repeated sequence which is present in a substantially fixed proportion in a genome contained in a sample to thereby obtain the number of cells in the sample; and using the number of cells as an index for normalizing a gene expression amount obtained from the sample.

For example, a DNA sample and an RNA sample are obtained from the same sample, the DNA sample is used as a sample for obtaining the number of cells, and the RNA sample is used as a sample for obtaining gene expression amounts, whereby the number of cells contained in the sample and the gene expression amounts relating to the same sample can be obtained. Therefore, the gene expression amounts obtained are converted into value per unit number of cells by use of the above-mentioned number of cells as an index, whereby the gene expression amounts can be normalized into values which can be compared with gene expression amounts obtained by other gene expression analyses.

To be more specific, for example, the measured value of hybridization of a probe nucleic acid for obtaining the number of cells which is immobilized on a substrate surface of a DNA chip with a target nucleic acid contained in the DNA sample is used as an index for normalizing the measured values of hybridization of probe nucleic acids for analysis of gene expression which are immobilized in other region on the substrate surface of the DNA chip with target nucleic acids contained in the RNA sample, whereby the gene expression amounts obtained can be normalized.

The above-mentioned repeated sequence may be obtained by searching for repeated sequences from fragments of genome information, or a sequence identical with an Alu sequence, which is a known repeated sequence, or with a part of the Alu sequence may be used as the repeated sequence.

Incidentally, the present invention can be systematized. In addition, in the above-mentioned method, the step of normalizing the numerical values relating to the gene expression amounts obtained from the same sample, by use of the numerical value relating to the number of cells in the sample which is obtained by measuring the repeated sequence present in a substantially fixed proportion in the genome, and a series of steps for searching for the repeated sequences from the fragments of genome information, can be automated by describing them in programs.

Definitions of terms used herein are as follows.

The term "repeated sequence" means a sequence such that the same base sequence is interspersed in a substantially fixed proportion in a genome, and the repeated sequence includes the sequences having the same base sequences as those of known repeated sequences (and parts thereof), such as SINE (Alu sequence, etc.) and LINE.

The term "gene expression amount" means the amount of expression of a specific gene in cells, and is a concept further including, for example, values measured through fluorescence intensity (measurement data) of the amounts of hybridization between probe nucleic acids immobilized on a substrate surface of a DNA chip and target nucleic acids capable of hybridization with the probe nucleic acids, and estimates of gene expression amounts obtained based on the measured values.

The term "normalization" means conversion of numerical values of fluorescence intensity or the like obtained by a gene expression analysis or the like into numerical values which can be compared with any measured values obtained by other gene expression analyses or the like.

The term "hybridization" means a reaction of forming a complementary strand (double strand) between nucleic acids which have complementary base sequence structures.

The term "nucleic acid" means a polymer (nucleotide strand) of a phosphate of nucleoside in which a purine or pyrimidine base and a sugar are combined by a glycosidic linkage; it widely includes DNAs (full length or fragments thereof) formed by polymerization of an oligonucleotide, polynucleotide, or purine nucleotide, including a probe DNA, with pyrimidine nucleotide, cDNA (complementary probe DNA) obtained by reverse transcription, RNA, polyamide nucleotide derivative (PNA), etc.

The term "probe nucleic acid" means a nucleic acid molecule which is present in a fixed or free state in a medium reserved or held in a reaction region and which functions as a probe for detecting a nucleic acid molecule having a complementary base sequence capable of a specific interaction therewith. Typical examples of the probe nucleic acid include oligonucleotides or polynucleotides, such as DNA probes. The term "target nucleic acid" means a nucleic acid which is one of sample nucleic acids sampled from cells and which is capable of hybridization with the probe nucleic acid.

According to the present invention, it is possible to enhance the accuracy in normalization of gene expression amounts.

### Brief Description of Drawings

[FIG. 1]

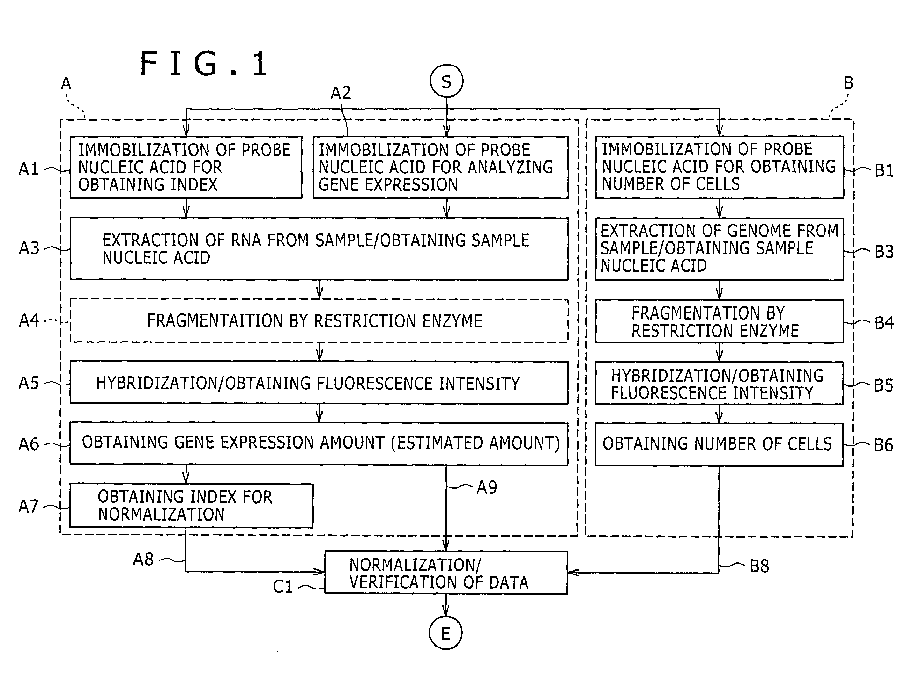

   FIG. 1 is a chart showing an example of the whole flow of normalization of gene expression amounts.
[FIG. 2]

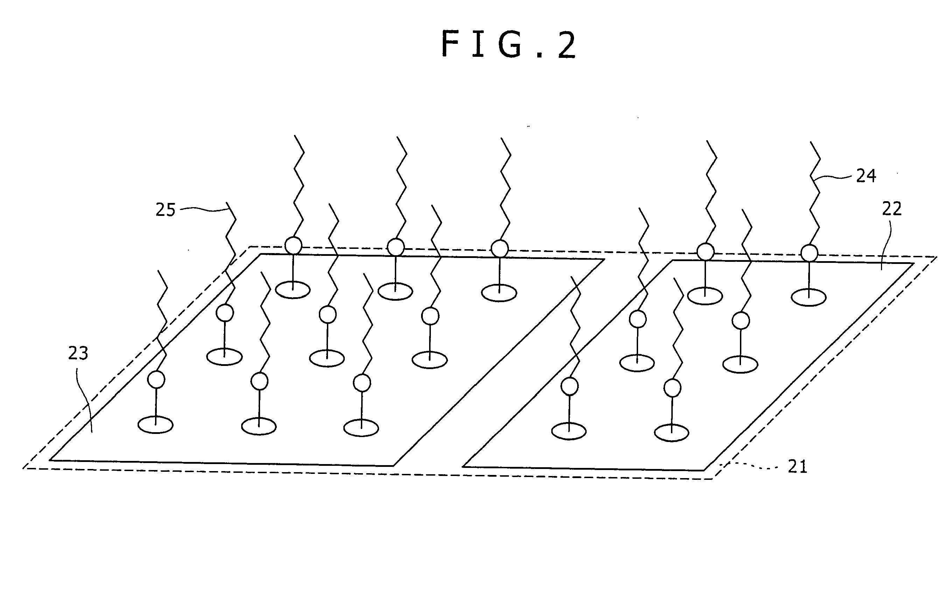

   FIG. 2 is a schematic diagram showing an example of a DNA chip used in the present invention.
[FIG. 3]

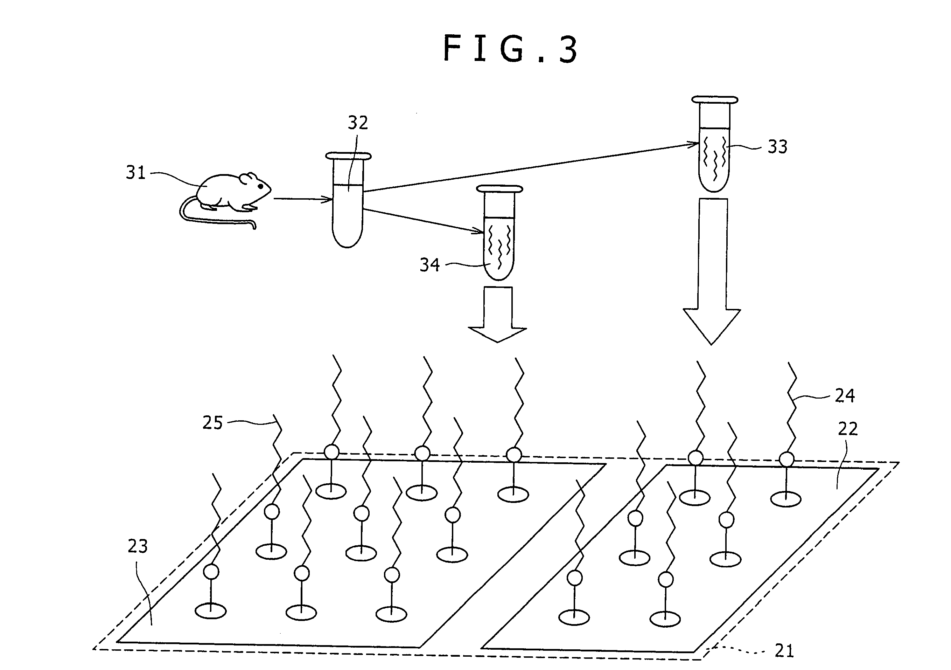

   FIG. 3 is a schematic diagram showing an example of the method of obtaining a DNA sample and an RNA sample from the same sample.
[FIG. 4]

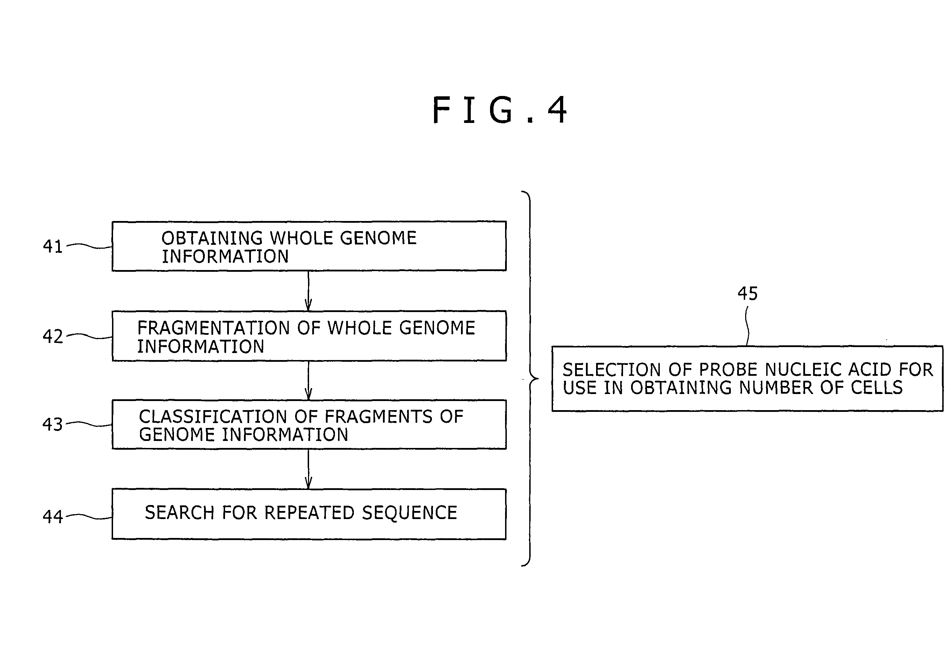

   FIG. 4 is a chart showing an example of the whole flow of research for repeated sequences in a genome.
[FIG. 5]

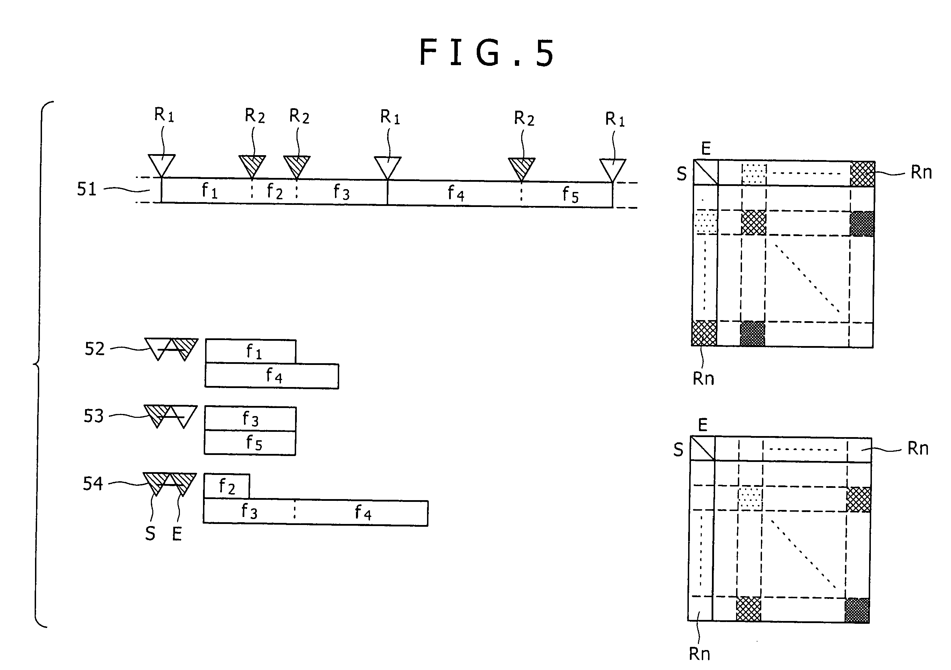

   FIG. 5 schematically shows a stage of fragmentizing whole genome information and a stage of classifying the fragments of genome information.
[FIG. 6]

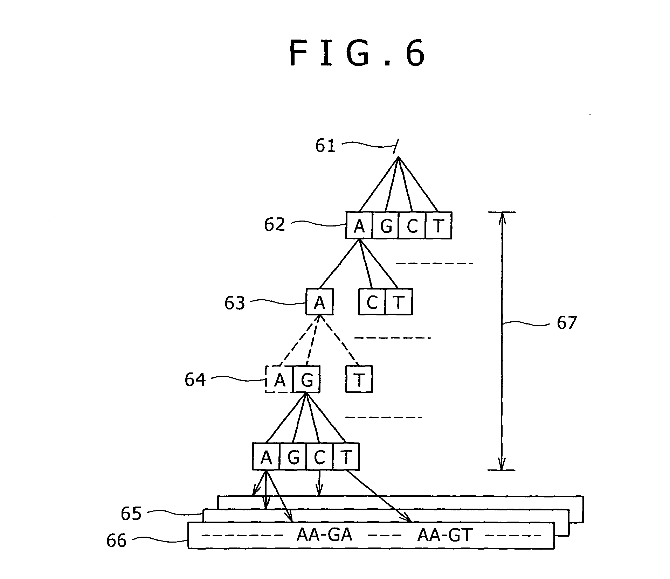

   FIG. 6 schematically shows a stage of searching for repeated sequences from the classified fragments of genome information.
[FIG. 7]

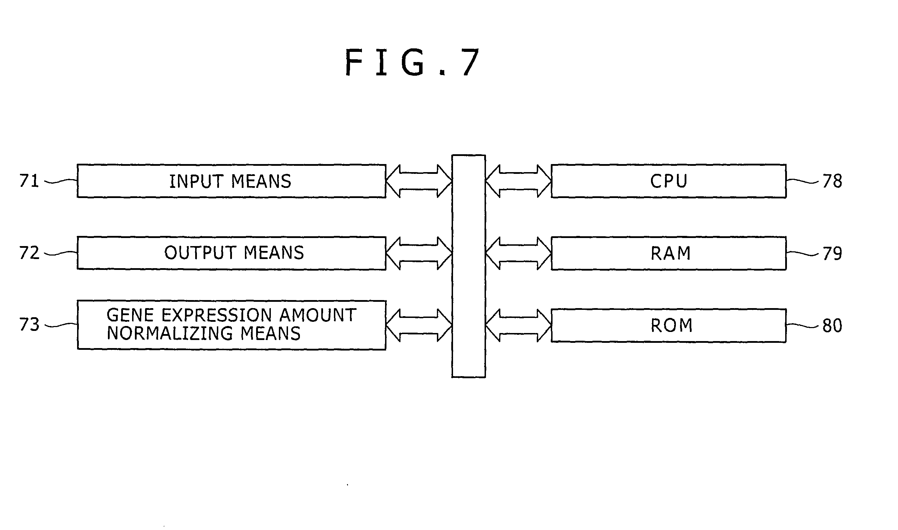

   FIG. 7 shows an example of the system according to the present invention.
[FIG. 8]

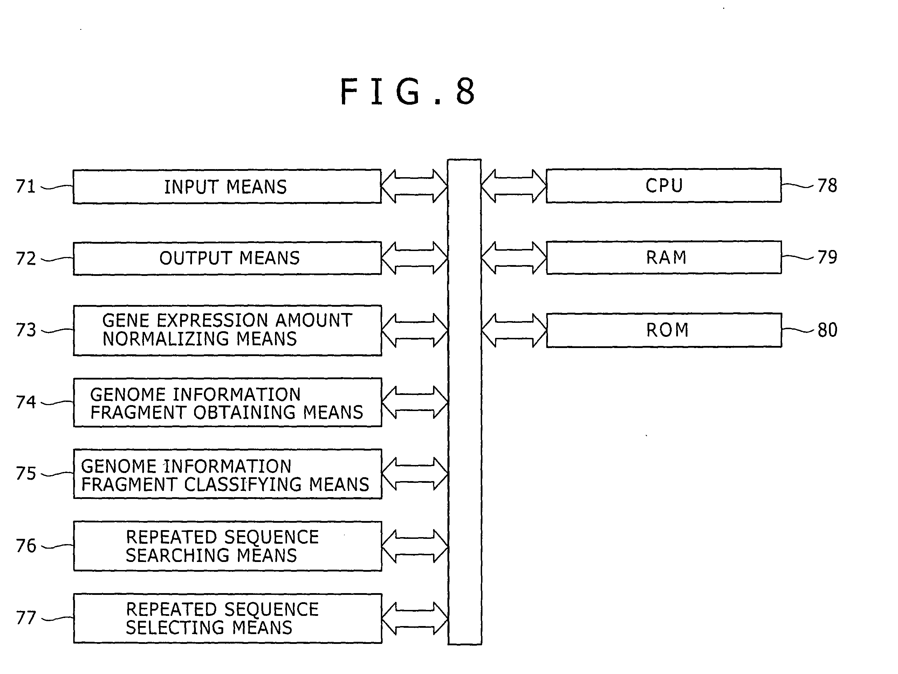

   FIG. 8 shows an example of the system according to the present invention.
[FIG. 9]
   FIG. 9 illustrates the related art, showing a method of using the gene expression amount of a gene expressed steadily as an index for normalization.

### Best Mode for Carrying out the Invention

Some preferred modes for carrying out the present invention will now be described below, referring to the accompanying drawings. Incidentally, the following embodiments exemplify the case where the amounts of hybridization between probe nucleic acids immobilized on a substrate surface of a DNA chip and target nucleic acids in sample nucleic acids obtained from a sample are obtained through fluorescence intensity, but the scope of the present invention is not to be narrowly construed thereby.

First of all, an example of the flow of normalization of gene expression amounts will be described referring to FIGS. 1 to 3.

FIG. 1 is a chart showing an example of the whole flow of normalization of gene expression amounts. In FIG. 1, flow B shows a genome processing flow, and flow A shows an RNA processing flow. Incidentally, in FIG. 1, "S" represents the starting point (START) of the flow, and "E" represents the ending point (END) of the flow.

The genome processing flow B is an example of the flow of obtaining the number of cells in a sample by measurement of a repeated sequence and using the thus obtained number of cells as an index for normalizing gene expression amounts obtained from the same sample. The number of repeated sequences present in a DNA sample correlates strongly with the amount of hybridization thereof. On the other hand, the repeated sequence is present in a substantially fixed proportion in a genome, so that the number of the repeated sequences present in the DNA sample strongly correlates also with the number of cells. Therefore, when a gene expression amount is normalized by use of the hybridization amount measured for obtaining the number of the repeated sequences, the dispersion of the gene expression amount generated due to the differences in the number of cells in the samples can be corrected. In short, by conversion of the gene expression amounts obtained into values per unit number of cells, it is possible to normalize the gene expression amounts to values which can be compared with those obtained by other gene expression analyses.

The genome processing flow B includes a stage (symbol B1) of preparing a DNA chip to be used in this flow, a stage (symbols B3 and B4) of obtaining and preparing sample nucleic acids from the sample obtained, and a stage (symbols B5 and B6) of measuring the amount of hybsridization between the probe nucleic acid for obtaining the number of cells and a target nucleic acid in the sample nucleic acids obtained from the sample through fluorescence, intensity and thereby obtaining the number of cells in the sample. These stages will be sequentially described below.

First, the stage (symbol B1) of preparing the DNA chip will be described. A probe nucleic acid for obtaining the number of cells is preliminarily immobilized on the substrate surface of the DNA chip to be used in the genome processing flow B. The probe nucleic acid for obtaining the number of cells contains, in an immobilized state, a nucleic acid for coding a repeated sequence (e.g., a sequence identical with an Alu sequence or a part thereof) present in a substantially fixed proportion in a genome. Incidentally, an example of the method of searching for the repeated sequence in a genome will be described later.

Next, the stage (symbols B3 and B4) of obtaining and preparing sample nucleic acids from the sample obtained will be described. In the genome processing flow B, according to the usual method, a genome DNA is extracted from the sample obtained, and the sample nucleic acids are obtained (symbol B3). The sample nucleic acids extracted from the genome DNA are fragmentized by restriction enzymes, before used (symbol B4).

Now, the stage (symbols B5 and B6) of measuring the amount of hybridization between the probe nucleic acid for obtaining the number of cells and the target nucleic acid in the sample nucleic acids obtained from the sample through fluorescence intensity and thereby obtaining the number of cells in the sample, will be described. The sample nucleic acids are supplied to the probe nucleic acid immobilized on the substrate surface of the DNA chip, and the amounts of hybridization between the probe nucleic acid and the target nucleic acid in the sample nucleic acids is measured by use of fluorescence intensity or the like (symbol B5). Then, the repeated sequence present in the target nucleic acid is quantitatively measured by use of fluorescence intensity or the like to thereby obtain the number of cells contained in the sample (symbol B6).

Then, gene expression amounts (arrow A9) based on the amounts of hybridization (measurement data) between a plurality of probe nucleic acids and target nucleic acids under gene expression analysis are converted into values per unit number of cells (arrow B8) by use of the number of cells contained in the sample as an index, whereby the gene expression amounts are normalized (symbol C1). Incidentally, this step can be automated by describing in the form of a program.

The RNA processing flow A includes a stage (symbol A1 and A2) of preparing a DNA chip to be used in this flow, a stage (symbols A3 and A4) of obtaining and preparing sample nucleic acids from the sample obtained, a stage (symbols A5 and A6) of measuring the amounts of hybridization between the probe nucleic acids immobilized on the substrate surface of the DNA chip and target nucleic acids in the sample nucleic acids obtained from the sample by use of fluorescence intensity to thereby obtain gene expression amounts, and a stage (symbol A7) of obtaining an index for normalizing the gene expression amounts measured. These stages will be sequentially described below.

First, the stage (symbols A1 and A2) of preparing the DNA chip will be described. The DNA chip to be used in the RNA processing flow A is preliminarily provided, in an immobilized state, with a plurality of probe nucleic acids for use in obtaining the index and probe nucleic acids for gene expression analysis. Incidentally, the immobilizing positions for the plurality of probe nucleic acids for use in obtaining the index are arbitrary; for example, the plurality of nucleic acids for use to obtain the index may be collectedly immobilized at a predetermined position on the substrate surface.

Next, the stage (symbols A3 and A4) of obtaining and preparing sample nucleic acids from the sample obtained will be described. In the RNA processing flow A, according to the usual method, RNA is extracted from the sample, and then the sample nucleic acids are obtained by, for example, synthesizing a cDNA having a sequence complementary to that of the RNA (symbol A3). The sample nucleic acids may be fragmentized by use of restriction enzymes (symbol A4).

Now, the stage (symbols A5 and A6) of measuring the amounts of hybridization between the probe nucleic acids immobilized on the substrate surface of the DNA chip and the target nucleic acids in the sample nucleic acids obtained from the sample by use of fluorescence intensity and thereby obtaining gene expression amounts, will be described. The sample nucleic acids are supplied to the probe nucleic acids immobilized on the substrate surface of the DNA chip, and the amounts of hybridization between the probe nucleic acids and the target nucleic acids in the sample nucleic acids are measured by use of fluorescence intensity or the like (symbol A5) . Then, based on the measurement data, the gene expression amounts (estimated amounts) are obtained (symbol A6).

Next, the stage (symbol A7) of obtaining the index for normalizing the gene expression amounts measured as above will be described. In the stage of symbol A7, a correlation among the plurality of gene expression amounts (symbol A6) measured for obtaining the index is obtained. Then, the correlation thus obtained is made to be the index for normalization of the gene expression amounts measured for gene analysis. This step can be automated by describing with a program. Here, the correlation means a value obtained from a correlation function in which the plurality of gene expression amounts measured for obtaining the index are used as parameters. The correlation function can be obtained, for example, by a method in which, as to a plurality of gene expression amounts obtained on a experimental condition basis from cells obtained respectively under two or more experimental conditions, the correlations among the plurality of gene expression amounts on the experimental condition basis are made to be function values, and such a combination that the function values are approximate to a fixed value is selected.

Then, by use of the index obtained in the stage of symbol A7 (arrow A8), the gene expression amounts (arrow A9) based on the amounts of hybridization (measurement data) between the plurality of probe nucleic acids for gene expression analysis and the target nucleic acids in the sample nucleic acids are normalized (symbol C1).

In addition to the above, by comparative examination of the gene expression amounts normalized based on the index obtained in the stage of symbol A7 and the gene expression amounts normalized based on the index obtained in the stage of symbol B6, verification of the measurement data can be performed (symbol C1). This step, also, can be automated by describing with a program.

FIG. 2 is a schematic diagram showing an example of the DNA chip used in the present invention (at the stage of symbol B1 in FIG. 1).

The substrate surface 21 of the DNA chip in FIG. 2 has a region 22 to be used for obtaining the number of cells and a region 23 to be used for gene expression analysis. In the region 22 for obtaining the number of cells, a probe nucleic acid 24 for obtaining the number of cells is immobilized, whereas in the region 23 for gene expression analysis, probe nucleic acids 25 for use in gene expression analysis are immobilized.

Incidentally, the probe nucleic acid 24 for obtaining the number of cells may be immobilized at any location on the substrate surface 21 of the DNA chip. Besides, while the region in which to immobilize the probe nucleic acid for obtaining the number of cells is provided on the substrate surface of the DNA chip for use in the RNA processing flow A, in FIG. 2, a DNA chip to be used for obtaining the number of cells may be prepared separately.

FIG. 3 is a schematic diagram showing an example of the method of obtaining a DNA sample and an RNA sample from the same sample (at the stage of symbols A3 and B3 in FIG. 1) .

A DNA sample 33 and an RNA sample 34 are obtained from a sample 32 obtained from an individual 31. The DNA sample 33 is obtained, for example, by extracting it from cells in the sample 32 according to a known method (symbol B3 in FIG. 1). Then, the thus obtained DNA sample 33 is dripped or supplied to the region 22 for obtaining the number of cells on the substrate surface 21 of the DNA chip, and the hybridization between the probe nucleic acid 24 for obtaining the number of cells and the target nucleic acid in the DNA sample is measured (symbol B5 in FIG. 1), to thereby obtain the number of cells (symbol B6 in FIG. 1).

On the other hand, the RNA sample 34 is obtained, for example, by extracting an RNA from the sample 32 and then synthesizing a cDNA having a sequence complimentary to that of the RNA according to a known method (symbol A3 in FIG. 1). Then, the thus obtained RNA sample 34 is dripped or supplied to the region 23 for gene expression analysis on the substrate surface 21 of the DNA chip, and hybridizations between the probe nucleic acids 25 for gene expression analysis and target nucleic acids in the RNA sample are measured (symbol A5 in FIG. 1), to thereby obtain gene expression amounts (symbol A6 in FIG. 1).

Now, the method of searching for repeated sequences in a genome will be described below, referring to FIG. 4 to 6.

In the case of obtaining the number of cells by the method according to the present invention, a known repeated sequence such as Alu sequence may be applied as the probe nucleic acid, or, alternatively, a sequence obtained as a result of search for a repeated sequence in a genome by the method described below may be applied as the probe nucleic acid.

FIG. 4 shows an example of the whole flow of search for a repeated sequence in a genome. The flow shown in FIG. 4 includes a stage (symbol 41) of obtaining the whole genome information, a stage (symbol 42) of fragmentizing the whole genome information, a stage (symbol 43) of classifying the fragments of genome information, and a stage (symbol 44) of searching for a repeated sequence from the classified fragments of genome information. Then, the repeated sequence obtained as a result of the search is selected as the probe nucleic acid to be used for obtaining the number of cells, and is immobilized on a substrate surface of a DNA chip (symbol 45). Incidentally, in relation to the stage (symbol 41) of obtaining the whole genome information, the whole genome information can be obtained from a known data base such as, for example, Gene Bank. In addition, since the whole genome information is huge in amount of information, it may be handled by dividing it on a chromosome basis.

FIG. 5 schematically shows the stage (symbol 42 in FIG. 4) of fragmentizing the whole genome information, and the stage (symbol 43 in FIG. 4) of classifying the fragmented genome information, i.e., the fragments of genome information.

First, the whole genome information 51 (or pieces of genome information divided on a chromosome basis) is searched for the recognition sequence(s) of one or a plurality of restriction enzymes R₁, R₂ ..., and is fragmentized at portions cleaved by the respective restriction enzymes R₁, R₂ .... Then, the fragmentized genome information, i.e., genome information fragments f₁, f₂ ... are obtained.

Next, the genome information fragments f₁, f₂ ... are classified on the basis of the restriction enzymes at both ends of each fragment which are associated with the fragmentization. For example, where fragmentization is carried out by the recognition sequences of two restriction enzymes R₁ and R₂, the genome information fragments f₁, f₂ ... can be classified into three kinds (symbols 52, 53, and 54) depending on the combination of the restriction enzyme (symbol S) related to the fragmentization on the N' terminal side of the genome information with the restriction enzyme (symbol E) related to the fragmentization on the C' terminal side. Similarly, where fragmentization is carried out by the recognition sequences of a plurality of restriction enzymes Rₙ, the restriction enzymes (symbol S) related to the fragmentization on the N' terminal side of the genome information and the restriction enzymes (symbol E) related to the fragmentization on the C' terminal side are arrayed respectively in a (vertical) column and in a (horizontal) row, as shown at the right in FIG. 5, whereby the genome information fragments can be classified.

FIG. 6 schematically shows the stage (symbol 44 in FIG. 4) of searching for a repeated sequence from the genome information fragments.

Since genome information is composed of four kinds A, G, C, and T, it is possible, by use of a tetrad 61 shown in FIG. 61, to find out whether or not an overlapping repeated sequence is present.

For example, in the case of FIG. 6, first, the genome information fragments classified by the above-mentioned method are searched for fragments having "A" (symbol 62). Next, among the thus searched genome information fragments having "A", the fragments having "A" at the next position of sequence are focused on (symbol 63). Then, the stepwise focusing (symbols 63 and 64) is sequentially repeated from the upstream side toward the downstream side of the tetrad 61, to find out the relevant genome information fragment (symbols 65 and 66). The number of times of search (symbol 67) is set to correspond to the length of the repeated sequence to be used as the probe nucleic acid. Then, after a predetermined number of times of stepwise focusing search, a combination of A, G, C, and T for which a multiplicity of genome information fragments have been searched is selected as the repeated sequence for use as the probe nucleic acid.

Now, an example of the system according to the present invention will be described below, referring to FIG. 7.

A gene expression amount normalizing system shown in FIG. 7 includes input means 71, an output means 72, gene expression amount normalizing means 73, a CPU 78, a RAM 79, and a ROM 80. The input means 71 is for inputting a numerical value relating to the number of cells in a sample, which value has been obtained by measuring a repeated sequence present in a substantially fixed proportion in a genome contained in the sample, and a function relating to normalization of the gene expression amount. The output means 72 is for outputting a function relating to normalization of the gene expression amount. The gene expression amount normalizing means 73 is for normalizing the gene expression amount by arithmetically processing the numerical value relating to the number of cells inputted by the input means, by use of the function.

Besides, a gene expression amount normalizing system shown in FIG. 8 includes: input means 71, output means 72, genome information fragment obtaining means 74, genome information fragment classifying means 75, repeated sequence searching means 76, repeated sequence selecting means 77, a CPU 78, a RAM 79, and a ROM 80, whereby repeated sequences in a genome can be searched for. The input means 71 is for inputting genome information. The output means 72 is for outputting a function relating to search for repeated sequences. The genome information fragment obtaining means 74 is for fragmentizing the genome information. The genome information fragment classifying means 75 is for classifying the genome information fragments. The repeated sequence searching means 76 is for searching for the repeated sequences from the classified fragments of genome information. The repeated sequence selecting means 77 is for selecting a repeated sequence to be used for obtaining the number of cells, from among the repeated sequences searched for.

### Industrial Applicability

According to the present invention, measured values of gene expression amounts or the like obtained by a gene expression analysis using a DNA chip or the like can be normalized and be enhanced in accuracy. In addition, measured values of hybridization can be normalized, so that respective measured values based on individual gene expression analyses can be compared and verified with high accuracy.

The method, program, and system according to the present invention can be easily incorporated into a measuring instrument such as a DNA chip.

## Claims

1. A method of normalizing a gene expression amount, comprising the steps of:
measuring a repeated sequence which is present in a substantially fixed proportion in a genome contained in a sample to thereby obtain the number of cells in said sample; and
using said number of cells as an index for normalizing a gene expression amount obtained from said sample.

2. The method of normalizing a gene expression amount as set force in claim 1, further comprising the steps of:
obtaining a DNA sample and an RNA sample from the same sample;
using said DNA sample as a sample for obtaining said number of cells; and
using said RAN sample as a sample for obtaining said gene expression amount.

3. The method of normalizing a gene expression amount as set forth in claim 2,
wherein a measured value of hybridization of a probe nucleic acid for obtaining said number of cells which is immobilized on a substrate surface of a DNA chip with a target nucleic acid contained in said DNA sample is used as an index for normalizing a measured value of hybridization of a probe nucleic acid for analyzing gene expression which is immobilized in another region on said substrate surface of said DNA chip with a target nucleic acid contained in said RNA sample.

4. The method of normalizing a gene expression amount as set forth in claim 1, wherein said repeated sequence is a sequence obtained by searching for a repeated sequence from fragments of genome information.

5. The method of normalizing a gene expression amount as set forth in claim 1, wherein said repeated sequence is a sequence identical with an Alu sequence or a part thereof.

6. A program for normalizing a gene expression amount, pertaining to a step of normalizing a numerical value related to a gene expression amount obtained from a sample by use of a numerical value related to the number of cells in said sample, said latter numerical value obtained by measuring a repeated sequence present in a substantially fixed proportion in a genome contained in said sample.

7. The program for normalizing a gene expression amount as set forth in claim 6, said program including programs related respectively to:
a step of fragmentizing genome information obtained;
a step of classifying said fragments of genome information;
a step of searching for repeated sequences from said fragments of genome information; and
a step of selecting a repeated sequence to be used for obtaining the number of cells, from among said searched repeated sequences.

8. A system for normalizing a gene expression amount, comprising at least:
input means for inputting a numerical value related to the number of cells in a sample, the numerical value obtained by measuring a repeated sequence which is present in a substantially fixed proportion in a genome contained in said sample, and a numerical value related to a gene expression amount obtained from said sample;
output means for outputting a function related to normalization of said gene expression amount; and
gene expression amount normalizing means for normalizing said gene expression amount by subjecting said numerical value related to said number of cells inputted by said input means to an arithmetic process with said function.

9. The system for normalizing a gene expression amount as set forth in claim 8, further comprising at least:
input means for inputting genome information;
output means for outputting a function related to search for a repeated sequence;
genome information fragment obtaining means for fragmentizing said genome information;
genome information fragment classifying means for classifying said fragments of said genome information;
repeated sequence searching means for searching for repeated sequences from said classified fragments of said genome information; and
repeated sequence selecting means for selecting a repeated sequence to be used for obtaining the number of cells, from among said searched repeated sequences.
